(19) Europäisches Patentamt
European Patent Office
Office européen des brevets

(11) Veröffentlichungsnummer : **0 331 905 B1**

# (12) EUROPÄISCHE PATENTSCHRIFT

(45) Veröffentlichungstag der Patentschrift : **03.06.92 Patentblatt 92/23**

(51) Int. Cl.$^5$ : **C07C 315/00**

(21) Anmeldenummer : **89101874.9**

(22) Anmeldetag : **03.02.89**

## (54) Verfahren zur Herstellung von 1,2-Disulfon-Verbindungen.

(30) Priorität : **12.02.88 DE 3804316**

(43) Veröffentlichungstag der Anmeldung : **13.09.89 Patentblatt 89/37**

(45) Bekanntmachung des Hinweises auf die Patenterteilung : **03.06.92 Patentblatt 92/23**

(84) Benannte Vertragsstaaten : **AT BE CH DE ES FR GB IT LI NL SE**

(56) Entgegenhaltungen : **ZEITSCHRIFT FÜT NATURFORSCHUNG, Band 21B, 1966, Seite 813; P. FLEMMING et al.: "Eine einfache Methode zur Darstellungaro-matischer 1.2-Disulfone"**

(73) Patentinhaber : **CIBA-GEIGY AG**
**Klybeckstrasse 141**
**CH-4002 Basel (CH)**

(72) Erfinder : **Bartmann, Ekkehard, Dr.**
**Dieburger Weg 12 a**
**W-6106 Erzhausen (DE)**

EP 0 331 905 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 1,2-Disulfon-Verbindungen.

1,2-Disulfon-Verbindungen besitzen ein vielfältiges Anwendungspotential. So ist beispielsweise aus Bull. Chem. Soc. Jap. 45, 2906 (1972) (CA 78: 15196c) bekannt, daß Diaryldisulfone photoreaktiv sind und unter Strahlungseinwirkung in Radikale zerfallen. Nach JP 58-83844 (CA 101: 63684a) werden solche Verbindungen als Radikalerzeuger in lichtempfindlichen Zusammensetzungen für photolithographische Zwecke eingesetzt. Gemäß Makromol. Chem., Rapid Commun. 4, 539 (1983) (CA 99: 140979v) und JP 59-197422 (CA 102: 186029u) können Diaryldisulfone als Strahlungsvernetzer für Epoxy-funtionalisierte Acrylatpolymere verwendet werden. Darüberhinaus sind 1,2-Disulfone wertvolle Reagenzien und Synthesezwischenprodukte, etwa auch bei der Synthese von pharmazeutischen Wirkstoffen.

Für 1,2-Disulfone mit im Prinzip beliebigen organischen Resten ist bislang kein Herstellverfahren bekannt, das bezüglich der Wahl der Substituenten in größtmöglicher Breite anwendbar und einfach durchzuführen ist. Diaryldisulfone sind gemäß JP 58-83844 durch Umsetzung von Aryl sulfinsäure-Alkalimetallsalzen mit Arylsulfonsäurechloriden zugänglich. Diese Methode ist aufwendig, im besonderen Maße bei unsymmetrisch substituierten Disulfonen. Die erzielbaren Produktausbeuten sind darüber hinaus unbefriedigend.

Ein vom Prinzip her einfacher Syntheseweg wird in Z. Naturforsch. 21b, 813 (1966) aufgezeigt. Dort wurden symmetrische Phenyl-, p-Tolyl- und Naphthyldisulfone durch Oxidation von entsprechenden Disulfonylhydrazinen hergestellt, wobei Ausbeuten zwischen etwa 30 und 50 % erzielbar waren. Als Oxidationsmittel fanden nur Quecksilberoxid und N-Bromsuccinimid Verwendung. Die Wahl dieser unüblichen, weil teueren, im Falle von Quecksilberoxid auch problematischen und alles in allem als "exotisch" zu bezeichnenden Oxidationsmittel legt den Schluß nahe, daß übliche Oxidationsmittel bei dieser Reaktion nicht zum Ziel führen.

Tatsächlich zeigte sich, daß praktisch alle üblichen Oxidationsmittel hierfür unbefriedigend oder gänzlich ungeeignet sind. Völlig überraschend wurde jedoch gefunden, daß konzentrierte Salpetersäure für diese Reaktion ein vorzüglich geeignetes Oxidationsmittel ist. Besonders unerwartet war hierbei, daß sich mit Hilfe von konzentrierter Salpetersäure neben symmetrischen und unsymmetrischen Diaryldisulfonen auch Aryl-Alkyldisulfone, Dialkyldisulfone sowie vielfältig substituierte Aryldisulfone und auch heteroaromatische Disulfone problemlos herstellen lassen.

Gegenstand der Erfindung ist daher ein Verfahren zur Herstellung von 1,2-Disulfon-Verbindungen durch Oxidation entsprechender 1,2-Disulfonylhydrazin-Verbindungen, wobei die Oxidation mit konzentrierter Salpetersäure als Oxidationsmittel durchgeführt wird.

Mit dem erfindungsgemäßen Verfahren lassen sich vielfältige 1,2-Disulfone mit weitgehend beliebigen organischen Resten darstellen. Insbesondere sind dies 1,2-Disulfon-Verbindungen der Formel I

$$R^1\text{-}SO_2\text{-}SO_2\text{-}R^2 \qquad \text{(I)}$$

worin $R^1$ und $R^2$ gleich oder verschieden sein können und Alkyl, Cycloalkyl, Aryl, Aralkyl oder Heteroaryl mit bis zu 12 C-Atomen, gegebenenfalls ein- oder mehrfach substituiert mit Halogen, Cyano, Nitro, Carboxyl, Alkyl, Alkoxy, Alkylthio, Bisalkylamino, Alkanoyl, Alkanoyloxy, Alkanoylamido, Alkoxycarbonyl, Alkylaminocarbonyl, Alkylsulfoxy, Alkylsulfonyl, Aryloxy, Arylthio, Arylsulfoxy, Arylsulfonyl mit jeweils bis zu 6 C-Atomen, bedeuten.

Die als Ausgangsprodukte einzusetzenden 1,2-Disulfonylhydrazin-Verbindungen sind in einfacher Weise nach bekannten Methoden durch Umsetzung von Hydrazin mit entsprechenden Sulfonylchloriden zugänglich, wobei durch stufenweise Umsetzung auch die unsymmetrischen 1,2-Disulfonylhydrazin-Verbindungen erhalten werden können.

Der gesamte Reaktionsablauf läßt sich in dem folgenden Reaktionsschema darstellen:

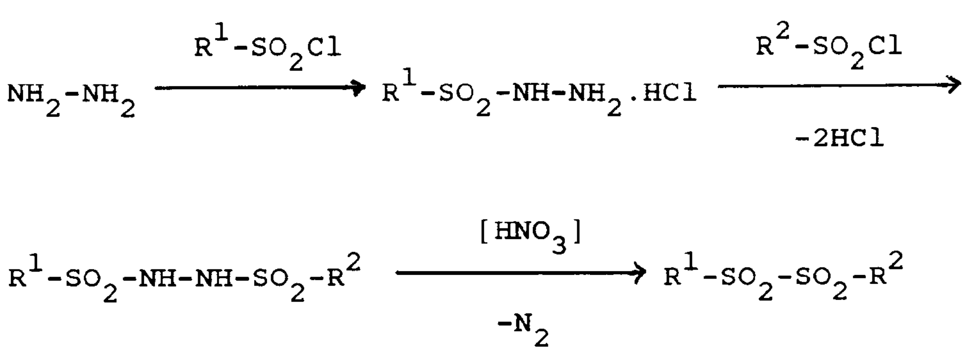


Die Oxidationsreaktion zum 1,2-Disulfon erfolgt erfindungsgemäß in der Weise, daß man zu der entsprechenden 1,2-Disulfonylhydrazin-Verbindung konzentrierte Salpetersäure von etwa der Dichte 1,4 und einer für Synthesezwecke üblichen Reinheit zufügt, zweckmäßigerweise unter Rühren und Kühlung. Da die Reaktionspartner unmittelbar miteinander zur Umsetzung gebracht werden können, ist die Anwesenheit eines Lösungsmittels entbehrlich. Die Temperatur des Reaktionsgemisches während der Zugabe und Umsetzung wird, etwa durch Eiskühlung des Reaktionsgefäßes im Bereich von -5 bis +5 °C, vorzugsweise bei ca. 0 °C gehalten. Die Reaktion setzt üblicherweise nach wenigen Minuten unter Stickstoffentwicklung ein. Das als Feststoffniederschlag ausgefallene oder nach Abschluß der Reaktion durch Zugabe von Wasser ausgefällte Produkt kann dann nach Bedarf durch Umkristallisation gereinigt werden.

In vielen Fällen ist es auch möglich die gesamte Reaktionsfolge, nämlich die Umsetzung von Hydrazin zu 1,2-Disulfonylhydrazin und die nachfolgende Oxidation zum 1,2-Disulfon in einer "Eintopfreaktion" ohne Isolierung der Disulfonylhydrazin-bzw. gegebenenfalls der Monosulfonylhydrazin-Zwischenstufe durchzuführen. Hierdurch wird das erfindungsgemäße Verfahren besonders einfach und wirtschaftlich.

Mit dem erfindungsgemäßen Oxidationsmittel lassen sich eine große Zahl unterschiedlichst substituierter 1,2-Disulfon-Verbindungen gut isolierbar und mit vorzüglicher bis zumindest zufriedenstellender Ausbeute erhalten. Im Gegensatz hierzu waren bei einer Vielzahl anderer üblicher Oxidationsmittel wie beispielsweise Wasserstoffperoxid, Kaliumpermanganat, Kaliumchlorat, Chlor oder Brom, um nur einige typische Vertreter herauszugreifen, entweder keine Reaktion zu verzeichnen, oder die Reaktion führte nicht oder nur in geringem Maße zum Disulfon. Lediglich mit Natriumhypochlorit gelang es in einigen wenigen. Fällen, nämlich bei Disulfonen der Formel I, in denen $R^1$ und $R^2$ Phenyl oder p-Tolyl sind, diese in mäßiger Ausbeute zu erhalten. In allen anderen Fällen versagte auch dieses Reagenz. Die Methode der Salpetersäure-Oxidation ist besonders günstig auch bei der Darstellung von Diaryldisulfonen, die empfindliche, das heißt zu Folgereaktionen neigende Substituenten an den Aryl-Resten tragen. Die Methode ist wegen ihrer vergleichsweise milden Bedingungen sogar für viele Substituenten verträglich, die mit anderen Oxidationsmitteln schnell oxidiert oder anderweitig umgewandelt werden. So bewirkt beispielsweise Natriumhypochlorit eine teilweise Chlorierung von i-Propyl-, Acetamido- und Dimethylmaleinimido-Resten am Aromaten, während Salpetersäure in diesen Fällen problemlos zum entsprechenden 1,2-Disulfon führt.

Beispiele

A. Allgemeine Vorschrift zur Herstellung von 1,2-Disulfonen der Formel $R^1$-$SO_2$-$SO_2$-$R^2$ (I)

Zur entsprechenden 1,2-Disulfonylhydrazinverbindung wird unter Rühren und Eiskühlung konzentrierte Salpetersäure (Dichte 1,4) getropft. Die Reaktion setzt nach einigen Minuten unter Stickstoffentwicklung ein. Nach Abklingen der Reaktion wird noch etwa 1 Stunde bei 0 °C gerührt, der ausgefallene Niederschlag abgetrennt und zur Reinigung umkristallisiert.

B. Hergestellte Verbindungen

| Nr. | $R^1$ | $R^2$ | Umkristallisiert aus | Schmelzpunkt (°C) |
|---|---|---|---|---|
| 1 | Phenyl | Phenyl | Ethanol | 192 |
| 2 | " | 4-Methylphenyl | Methanol | 177 |
| 3 | " | 4-Methoxyphenyl | Aceton | 153 |
| 4 | " | 2-Naphthyl | Methyl-t-Butylether | 182 |
| 5 | " | Benzyl | Methyl-t-Butylether/Aceton | 186 |
| 6* | " | 2-Nitro-3,5-dimethoxyphenyl | Aceton | 186 |
| 7 | " | 2,4,6-Trimethylphenyl | Toluol | 154 |
| 8 | " | 4-i-Propylphenyl | Eisessig | 92 |
| 9 | " | 4-Chlorphenyl | Toluol | 181 |
| 10 | " | 4-Bromphenyl | Aceton | 198 |
| 11 | 4-Methylphenyl | 4-Methylphenyl | Aceton/Ether | 222 |
| 12 | " | 4-Chorphenyl | Toluol | 206 |
| 13 | " | Benzyl | Methanol | 126 |

| | | | | |
|---|---|---|---|---|
| 14 | 4-Methylphenyl | 2-Naphthyl | Methyl-t-Butylether/Dichlormethan | 186 |
| 15 | " | 4-Methoxyphenyl | Aceton | 173 |
| 16* | " | 2-Nitro-3,5-dimethoxyphenyl | Aceton | 182 |
| 17 | " | Methyl | Methyl-t-Butylether | 109 |
| 18 | " | 1-Naphthyl | Aceton | 201 |
| 19 | 1-Naphthyl | 1-Naphthyl | Aceton/DMF | 183 (Zersetzung) |
| 20 | 2-Naphthyl | 2-Naphthyl | THF | 226 (Zersetzung) |
| 21 | 4-Methoxyphenyl | 4-Methoxyphenyl | Aceton | 194 (Zersetzung) |
| 22 | 4-Nitrophenyl | 4-Nitrophenyl | THF | 224 (Zersetzung) |
| 23 | 2-Methylphenyl | 2-Methylphenyl | Toluol | 160 |
| 24 | Benzyl | Benzyl | Eisessig | 183 (Zersetzung) |
| 25 | Benzyl | n-Propyl | Dichlormethan/n-Pentan | 100 |
| 26 | n-Propyl | n-Propyl | (Wasser)** | 53 |
| 27 | n-Propyl | 4-Nitrophenyl | Methyl-t-Butylether | 111 |
| 28 | n-Propyl | 4-Methoxyphenyl | Petrolether | 83 |
| 29 | Phenyl | n-Propyl | Methyl-t-Butylether | 138 |
| 30 | " | 4-t-Butylphenyl | (Wasser)** | 128 |
| 31 | 4-Methylphenyl | n-Propyl | (Wasser)** | 86 |
| 32 | 4-i-Propylphenyl | Methyl | - | (Öl) |

| | | | | |
|---|---|---|---|---|
| 33 | 4-t-Butyl-phenyl | n-Propyl | Ether/Petrol-ether | 110 |
| 34 | 1-Naphthyl | Methyl | Toluol | 157 |
| 35 | 1-Naphthyl | n-Propyl | (Wasser)** | 116 |
| 36 | 2-Naphthyl | Methyl | (Wasser)** | 146 |
| 37 | 2-Naphthyl | n-Propyl | Ether/Petrolether | 67 |
| 38 | 4-Acetylamido-phenyl | n-Propyl | Eisessig | 189 |
| 39 | " | Phenyl | " | 208 |
| 40 | " | 4-Methylphenyl | " | 201 |
| 41 | " | 4-Methoxyphenyl | " | 204 |
| 42 | " | 4-Nitrophenyl | " | 198 (Zer-setzung) |
| 43 | 4-Phthalimido-phenyl | n-Propyl | " | 196 |
| 44 | " | Phenyl | Dichlormethan/ Cyclohexan | 210 |
| 45 | " | 4-Methylphenyl | Eisessig | 214 |
| 46 | " | 4-Methoxyphenyl | " | 162 |
| 47 | 4-(1,2-Di-methylmalein-imido)phenyl | n-Propyl | Methyl-t-butylether | 143 |
| 48 | " | Phenyl | Dichlormethan | 222 |
| 49 | " | 4-Methylphenyl | Dichlormethan/ Petrolether | 235 |
| 50 | " | 4-Methoxyphenyl | Eisessig | 162 |
| 51 | 4-Nitrophenyl | n-Propyl | Ether | 104 |

* Die Einführung der $NO_2$-Gruppe erfolgte hier während der Salpetersäure-Oxidation

30

** Kristallisiert bereits bei Zugabe von Wasser in ausreichender Reinheit aus

C. Vergleichende Oxidationsversuche

Zu einigen der in B. hergestellten 1,2-Disulfon-Verbindungen wurde im Vergleich mit der Methode der Oxidation mit konzentrierter Salpetersäure die Eignung anderer Oxidationsmittel getestet. Der Einsatz der anderen Oxidationsmittel erfolgte nach den für diese üblichen Reaktionsbedingungen.

In der nachfolgenden Tabelle 1 bedeutet:

‡ Disulfon in guter Ausbeute isolierbar

- \+ Disulfon in mäßiger Ausbeute isolierbar
- o Disulfon in geringer Menge nachweisbar, jedoch nicht isolierbar
- \- Disulfon nicht nachweisbar; andere Reaktionsprodukte
- = keine Reaktion

Tabelle 1: Vergleichende Oxidationsversuche

| Verbindung Nr. | 1 | 2 | 8 | 11 | 13 | 24 | 25 | 29 | 31 | 32 | 33 | 36 | 37 | 41 | 44 |
|---|---|---|---|---|---|---|---|---|---|---|---|---|---|---|---|
| $HNO_3$ | + + | + + | + + | + + | + | + | + | + + | + + | + + | + + | + + | + + | + + | + + |
| $NaOCl/H_2O$ | + | + | o | + | - | - | | o | o | - | o | - | - | - | + |
| $H_2O_2/Na_2WO_4$ | = | | | | | | | | | | | | | | |
| $K_2S_2O_8/H_2SO_4$ | | | | | | = | | | | | | | | | |
| N-Bromsuccin-imid/$CH_2Cl_2$ | | | | | | - | | - | | | | | | | |
| $K_3Fe(CN)_6$ | | = | | | | | | | | | | | | | |
| Benzoylperoxid/ Chlorbenzol | | | | = | | | | | | | | | | | |
| Chlorkalk | | | | = | | | | | | | | | | | |
| $Ce^{IV}/H_3O^+$ | - | | | | | | | | | | | | | | |
| $KClO_3/H_3O^+$ | = | | | | | | | | | | | | | | |
| $KMnO_4$/Aceton | | | | = | | | | | | | | | | | |
| $KMnO_4/CH_3CO_2H$ | | | | + | - | | | o | | | | | | | |
| m-Chlorperben-zoesäure/ $CH_2Cl_2$ | + | | | | | - | - | o | | | | | | | |
| $Cl_2/H_2SO_4$ | | | | - | | | | | | | | | | | |
| $Cl_2/CCl_4$ | | | | - | | | | - | | | | | | | |
| $Br_2/C_2H_5OH$ | | | | o | | | | | | | | | | | |
| $Br_2/H_2SO_4$ | | | | | | - | - | | | | | | | | |
| $Br_2/CH_3CO_2H$ | - | | | o | - | - | - | | | | | | | | |
| $H_2O_2/HCl/H_2O$ | | | | = | | | | | | | | | | | |
| $H_2O_2/HCO_2H$ | = | | | | | | | | | | | | | | |
| $H_2O_2/CH_3CO_2H$ | | | | = | | | | | | | | | | | |

## Patentansprüche

1. Verfahren zur Herstellung von 1,2-Disulfon-Verbindungen durch Oxidation entsprechender 1,2-Disulfonylhydrazin-Verbindungen, dadurch gekennzeichnet, daß die Oxidation mit Hilfe von konzentrierter Salpetersäure als Oxidationsmittel durchgeführt wird.

2. Verfahren nach Anspruch 1, dadurch gekennzeichnet, daß die Reaktionspartner unmittelbar und bei einer Temperatur zwischen -5 und +5 °C zur Umsetzung gebracht werden.

## Claims

1. Process for the preparation of 1,2-disulfone compounds by oxidation of corresponding 1,2-disulfonylhydrazine compounds, characterized in that the oxidation is carried out with the aid of concentrated nitric acid as the oxidizing agent.

2. Process according to Claim 1, characterized in that the reaction partners are reacted with one another directly and at a temperature between -5 and +5°C.

## Revendications

1. Procédé de préparation de 1,2-disulfones par oxydation de 1,2-disulfonylhydrazines correspondantes, caractérisé en ce qu'on conduit l'oxydation à l'aide d'acide nitrique concentré comme oxydant.

2. Procédé selon la revendication 1, caractérisé en ce que les réactifs sont mis à réagir immédiatement et à une température comprise entre -5 et +5°C.